# EUROPEAN PATENT SPECIFICATION

(11) **EP 2 639 303 B1**
(45) Date of publication and mention of the grant of the patent: **07.06.2017**
(21) Application number: 11839803.1
(22) Date of filing: 08.11.2011
(51) Int. Cl.: C12N 15/09, C12N 5/10

(54) **NOVEL EXPRESSION VECTOR**
NEUER EXPRESSIONSVEKTOR
NOUVEAU VECTEUR D'EXPRESSION

(30) Priority: 08.11.2010 JP 2010250306
(43) Date of publication of application: 18.09.2013
(73) Proprietor: JCR Pharmaceuticals CO., LTD., Ashiya-shi, Hyogo 659-0021 (JP)
(72) Inventor: TAKAHASHI, Kenichi, Kobe-shi Hyogo 651-2241 (JP)
(74) Representative: Isarpatent
(86) International application number: PCT/JP2011/075670
(87) International publication number: WO 2012/063799

(56) References cited:
- WO-A1-01/55371
- WO-A1-2010/097240
- WO-A2-02/094989
- CN-A- 101 597 616
- PU H ET AL: "RAPID ESTABLISHMENT OF HIGH-PRODUCING CELL LINES USING DICISTRONIC VECTORS WITH GLUTAMINE SYNTHETASE AS THE SELECTION MARKER", MOLECULAR BIOTECHNOLOGY, HUMANA PRESS, INC, US, vol. 10, no. 1, 1 August 1998 (1998-08-01) , pages 17-25, XP009021044, ISSN: 1073-6085, DOI: 10.1007/BF02745860
- "BICISTRONIC VECTOR FOR THE CREATION OF STABLE MAMMALIAN CELL LINES THAT PREDISPOSES ALL ANTIBIOTIC-RESISTANT CELLS TO EXPRESS RECOMBINANT PROTEIN", BIOTECHNIQUES, INFORMA HEALTHCARE, US, vol. 20, no. 1, 1 January 1996 (1996-01-01), XP001093737, ISSN: 0736-6205
- DAVIES M V ET AL: "Internal translation initiation in the design of improved expression vectors", CURRENT OPINION IN BIOTECHNOLOGY, LONDON, GB, vol. 3, no. 5, 1 October 1992 (1992-10-01) , pages 512-517, XP023601259, ISSN: 0958-1669, DOI: 10.1016/0958-1669(92)90079-X [retrieved on 1992-10-01]
- DOUIN VICTORINE ET AL: "Use and comparison of different internal ribosomal entry sites (IRES) in tricistronic retroviral vectors", BMC BIOTECHNOLOGY, BIOMED CENTRAL LTD. LONDON, GB, vol. 4, no. 1, 27 July 2004 (2004-07-27), page 16, XP021005932, ISSN: 1472-6750, DOI: 10.1186/1472-6750-4-16
- MARTIN, PATRICK ET AL.: 'Development of a new bicistronic retroviral vector with strong IRES activity.' BMC BIOTECHNOL. vol. 6, no. 4, 2006, pages 1 - 9, XP021017066
- PU, HAIFENG ET AL.: 'Rapid establishment of high-producing cell lines using dicistronic vectors with glutamine synthetase as the selection marker.' MOL. BIOTECHNOL. vol. 10, no. 1, 1998, pages 17 - 25, XP009021044
- TROUET, DOMINIQUE ET AL.: 'Use of a bicistronic GFP-expression vector to characterise ion channels after transfection in mammalian cells.' PFLUGERS ARCH. vol. 434, no. 5, 1997, pages 632 - 638, XP009069058
- HAVENGA, M.J.E. ET AL.: 'Second gene expression in bicistronic constructs using short synthetic intercistrons and viral IRES sequences.' GENE vol. 222, no. 2, 1998, pages 319 - 327, XP027360761
- VAN BLOKLAND, H.J.M. ET AL.: 'A novel, high stringency selection system allows screening of few clones for high protein expression.' J. BIOTECHNOL. vol. 128, 2007, pages 237 - 245, XP005829332
- DAVIES, MONIQUE V. ET AL.: 'The sequence context of the initiation codon in the encephalomyocarditis virus leader modulates efficiency of internal translation initiation.' J.VIROL. vol. 66, no. 4, 1992, pages 1924 - 1932, XP055087053
- BOCHKOV, YURY A. ET AL.: 'Translational efficiency of EMCV IRES in bicistronic vectors is dependent upon IRES sequence and gene location.' BIOTECHNIQUES vol. 41, no. 3, 2006, pages 283 - 290, XP001536676
- MENG, Y. GLORIA ET AL.: 'Green fluorescent protein as a second selectable marker for selection of high producing clones from transfected CHO cells.' GENE vol. 242, 2000, pages 201 - 207, XP004196520
- WIRTH, M. ET AL.: 'Isolation of overproducing recombinant mammalian cell lines by a fast and simple selection procedure.' GENE vol. 73, 1988, pages 419 - 426, XP025705564

## Description

### TECHNICAL FIELD

The present invention relates to a novel expression vector for efficient expression of recombinant proteins in mammalian cells, in particular to an expression vector which comprises a gene expression regulatory site, a gene encoding a protein of interest downstream thereof, an internal ribosome entry site further downstream thereof, and a gene encoding a glutamine synthetase still further downstream thereof.

### BACKGROUND ART

In some fields of industry such as drug manufacturing, a familiar technology is a method for production of a recombinant protein of interest using mammalian cells which is transformed with an expression vector containing an incorporated gene encoding the protein. Using this technology, various products are produced and marketed, e.g., lysosomal enzymes such as α-galactosidase A, iduronate-2-sulfatase, glucocerebrosidase, galsulfase, α-L-iduronidase, α-glucosidase, and the like; tissue plasminogen activator (t-PA); blood coagulation factors such as blood coagulation factor VII, blood coagulation factor VIII, blood coagulation factor IX, and the like; erythropoietin; interferon; thrombomodulin; follicle-stimulating hormone; granulocyte colony-stimulating factor (G-CSF); various antibody medicaments, and the like.

In performing this technology, it is a general practice to employ an expression vector in which a gene encoding a protein of interest is incorporated downstream of a gene regulatory site that induces a potent expression of a gene, such as a cytomegalovirus (CMV)-derived promoter, SV40 early promoter, or elongation factor 1α (EF-1) promoter. Mammalian cells, after introduction therein of such an expression vector, come to express the protein of interest incorporated in the expression vector. The levels of its expression, however, vary and are not even among the cells. Therefore, for efficient production of the recombinant protein, a step is required to select, from the mammalian cells having the expression vector introduced therein, those cells which express the protein of interest at high levels. For performing the selection step, a gene which acts as a selection marker is incorporated in an expression vector.

The most popular of such selection markers are enzymes (drug resistance markers) which decompose drugs such as puromycin, neomycin, and the like. Mammalian cells will be killed in the presence of these drugs over certain concentrations. Mammalian cells into which an expression vector has been introduced, however, become viable in the presence of those drugs because such cells can decompose the drugs with the drug selection markers incorporated in the expression vector and thus detoxify them or weaken their toxicity. Therefore, when those cells having such an incorporated expression marker are cultured in a medium containing a corresponding drug mentioned above, over a certain concentration, only such cells grow that express the corresponding selection marker at high levels, and as a result, they are selected. Such cells which express a drug selection marker at high levels also tend to express, at high levels, a gene encoding a protein of interest incorporated together in the expression vector, and as a result, mammalian cell thus will be obtained which express the protein of interest at high levels.

Expression vectors are also known in which a glutamine synthetase (GS) is used as a selection marker (cf. Patent Documents 1 and 2). Glutamine synthetase is an enzyme which synthesizes glutamine from glutamic acid and ammonia. If mammalian cells are cultured in a medium which lacks glutamine in the presence of methionine sulfoximine (MSX), an inhibitor of glutamine synthetase, at a certain concentration, the cells will be annihilated. However, if an expression vector in which a glutamine synthetase is incorporated as a selection marker is introduced into mammalian cells, the cells, now with increased levels of expression of the glutamine synthetase, become able to grow even in the presence of higher concentrations of MSX. In doing this, if culture is continued with a gradually increasing concentration of MSX, such cells are obtained that can grow in the presence of still higher concentrations of MSX. This phenomenon is thought to be brought about by multiplication, in number in the genome, of the expression vector incorporated in the genome of the mammalian cells. Namely, along with an increasing number of the copies, the number of the gene for the drug selection marker also increases in number in the genome of the cell, resulting in relative elevation of the expression levels of the gene per cell. In this situation, as the gene encoding the protein of interest incorporated in the expression vector is also multiplied and its copy number increased, such mammalian cells are obtained that express the protein of interest at high levels. For example, Patent Document 1 discloses that employment of a GS expression vector and methionine sulfoximine (MSX) allows achievement of an increase of the copy numbers which is higher than those achieved by using DHFP (dihydrofolate reductase)/MTX (methotrexate). Further, Patent Document 2 discloses that by employment of a GS gene and MSX, copy numbers of a different, heterozygous gene can also be increased, along with increased numbers of copies of the GS gene, which thereby enables increased production levels of a polypeptide of interest.

Thus, expression vectors containing a selection marker are suitable for efficient production of recombinant proteins, and thus are commonly used. A gene encoding a protein of interest and a gene encoding a selection marker are generally incorporated in an expression vector downstream of respective different gene regulatory sites (cf. Patent Document 3). However, a method is also known in which genes encoding a protein of interest and a selection marker are incorporated in series downstream of a single gene regulatory site to let them express themselves (cf. Patent Documents 4, 5, 6, and 7). In performing this, an internal ribosome entry site (IRES) and the like are inserted between the genes encoding a protein of interest and a selection marker, which enables expression of two genes under a single gene regulatory site. Various internal ribosome entry sites are known: for example, those derived from picornavirus, poliovirus, encephalomyocarditis virus, and chicken infectious Fabricius bursal disease virus (cf. Patent Documents 8, 9, and 10).

Among expression vectors utilizing an internal ribosome entry site, there are known an expression vector in which herpes simplex virus thymidine kinase is incorporated as a selection marker downstream of an internal ribosome entry site (cf. Patent Document 11), and an expression vector in which three or more genes are combined using two or more internal ribosome entry sites (cf. Patent Document 12).

As mentioned above, owing to development of various expression vectors, methods for production of recombinant proteins using mammalian cells have been in practical use for production of medicaments, such as erythropoietin and the like. However, development of expression vectors which are more efficient than conventional ones are consistently sought in order to lower the cost for their production.

### [Prior Art Documents]

### [Patent Documents]

[Patent Document 1] Japanese Patent Application Publication No. S63-502955
[Patent Document 2] Japanese Patent Application Publication No. H05-504050
[Patent Document 3] Japanese Patent Application Publication No. 2009-273427
[Patent Document 4] Japanese Patent Application Publication No. S59-173096
[Patent Document 5] Japanese Patent Application Publication No. S60-19938 7
[Patent Document 6] Japanese Patent Application Publication No. H04-500004
[Patent Document 7] Japanese Patent Application Publication No. H08-256776
[Patent Document 8] Japanese Patent Application Publication No. H06-509713
[Patent Document 9] Japanese Patent Application Publication No. H08-502644
[Patent Document 10] Japanese Patent Application Publication No. H10-327871
[Patent Document 11] Japanese Patent Application Publication No. 2008-539785
[Patent Document 12] Japanese Patent Application Publication No. 2004-520016

### SUMMARY OF INVENTION

### [Problem to be Solved by Invention]

The objectives are to provide a novel expression vector for efficient expression of recombinant proteins in mammalian cells, mammalian cells transformed with the vector, and a method for production of such mammalian cells.

### [Means to Solve the Problem]

In a study directed to the above objectives, the present inventors transformed mammalian cells with an expression vector in which are incorporated an gene expression regulatory site, and a gene encoding a protein of interest, such as human glucocerebrosidase, downstream thereof, an internal ribosome entry site further downstream thereof, and a gene encoding glutamine synthetase still further downstream thereof, and found that a high level expression of the gene encoding the protein thereby becomes available, having completed the present invention.

### EFFECT OF INVENTION

According to the present invention, an expression vector is provided for efficient expression of a recombinant protein of interest in mammalian cells. Transformed cells which efficiently produce a recombinant protein can be obtained by introducing the expression vector into mammalian cells and then subjecting the cells to a selective culture. Use of thus obtained transformed cells enables significant cost reduction in the production of recombinant proteins.

### BRIEF DESCRIPTION OF DRAWINGS

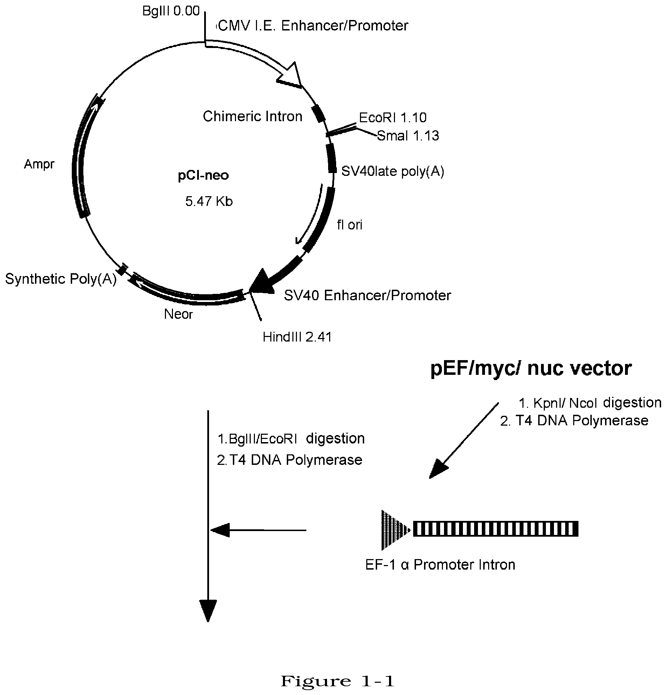

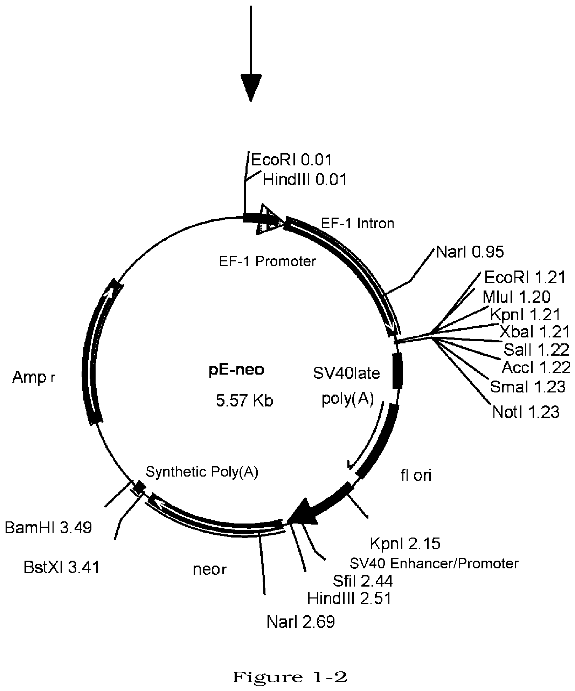

[Fig. 1-1] A diagram illustrating a flow of the method for construction of pE-neo vector.
[Fig. 1-2] A diagram illustrating a flow of the method for construction of pE-neo vector.
[Fig. 2-1] A diagram illustrating a flow of the method for construction of pE-hygr vector.
[Fig. 2-2] A diagram illustrating a flow of the method for construction of pE-hygr vector.

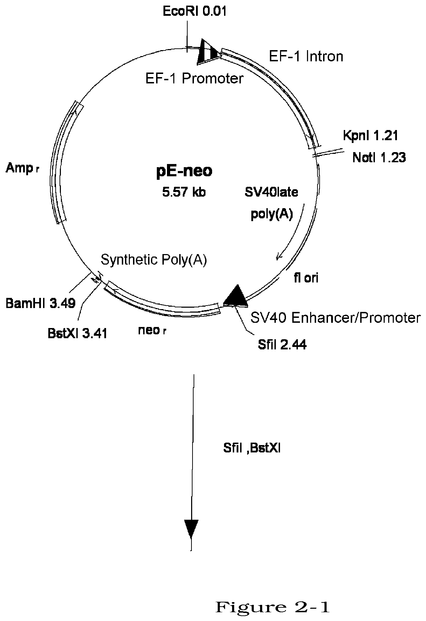

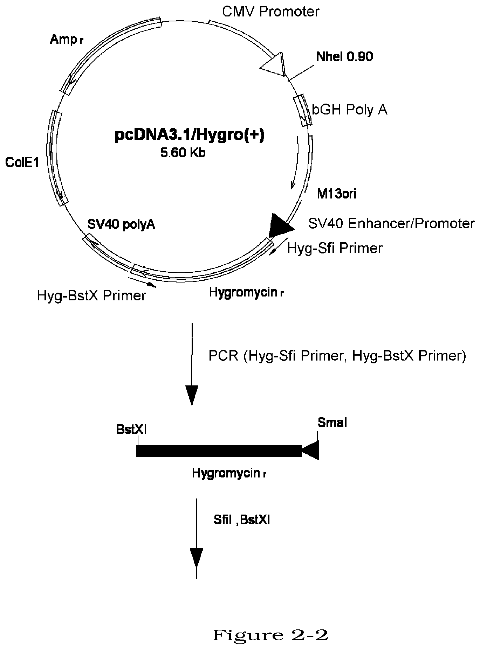

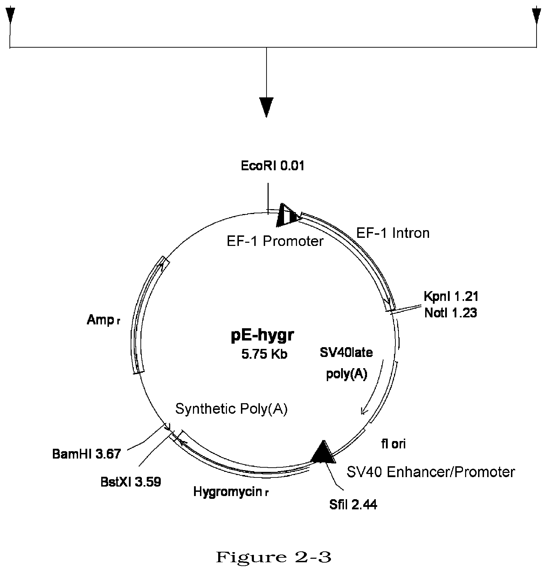

[Fig. 2-3] A diagram illustrating a flow of the method for construction of pE-hygr vector.

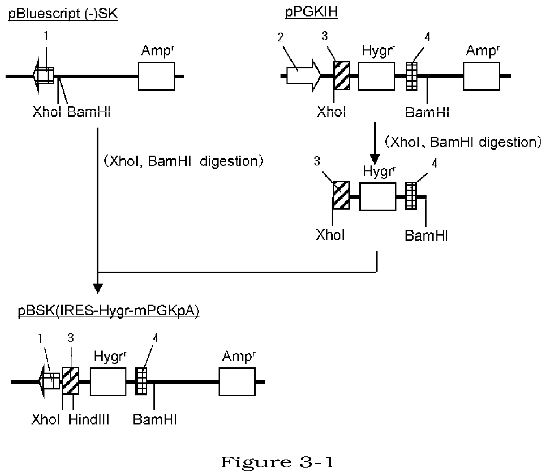

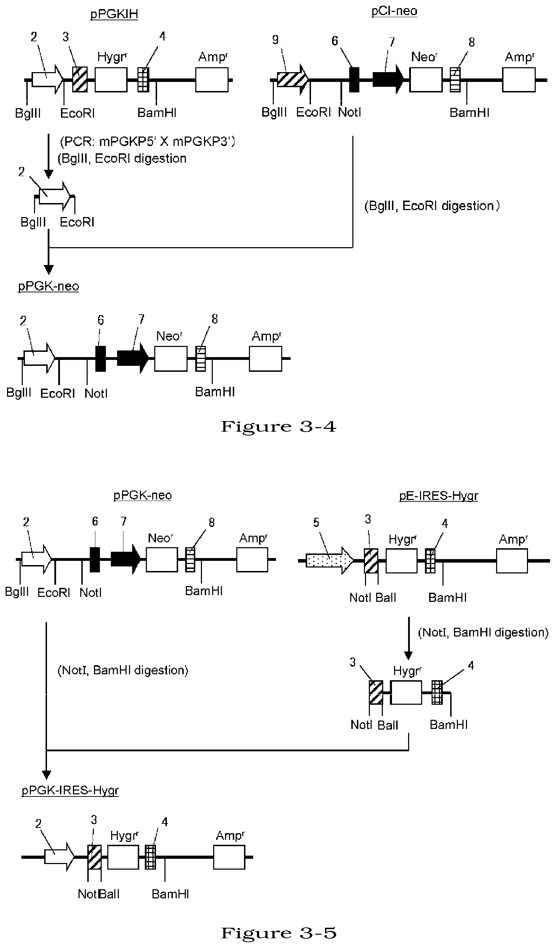

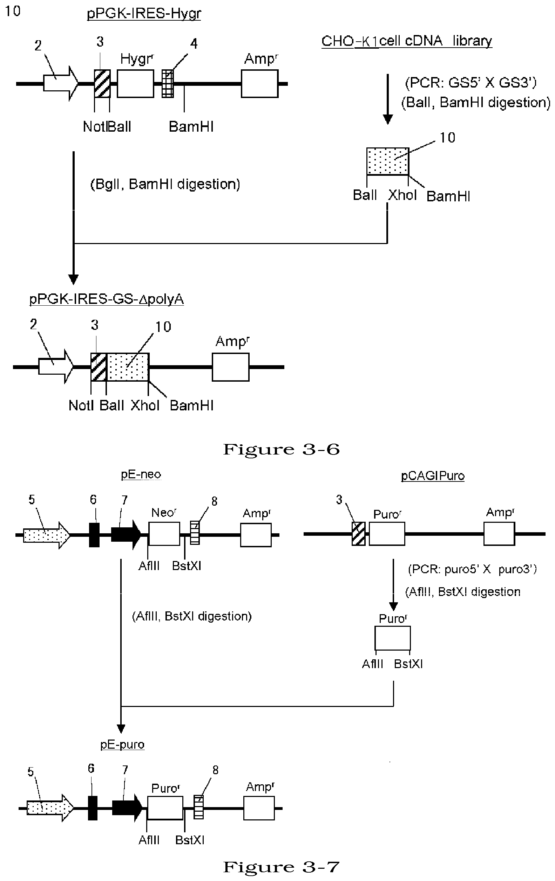

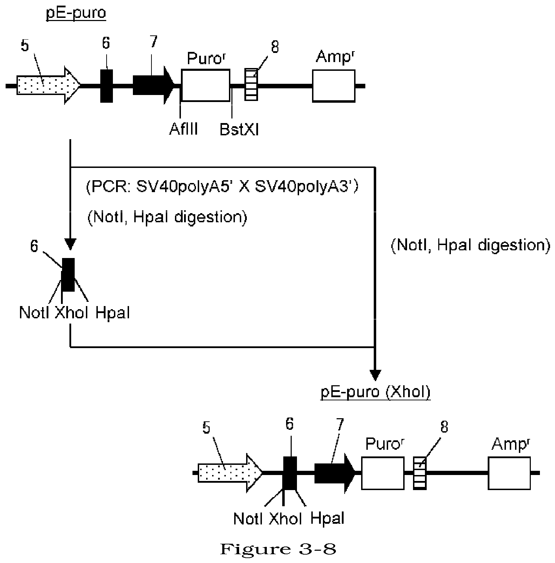

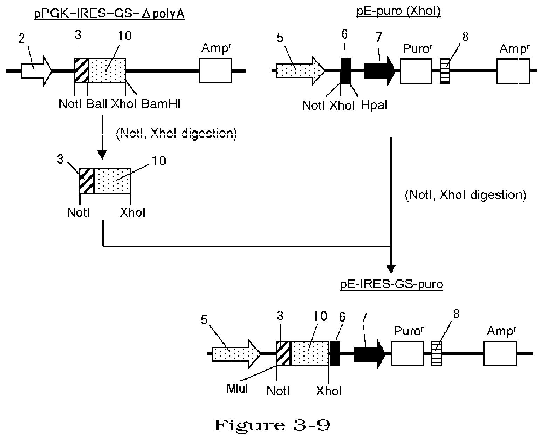

[Fig. 3-1] A diagram illustrating a flow of the method for construction of pE-IRES-GS-puro.

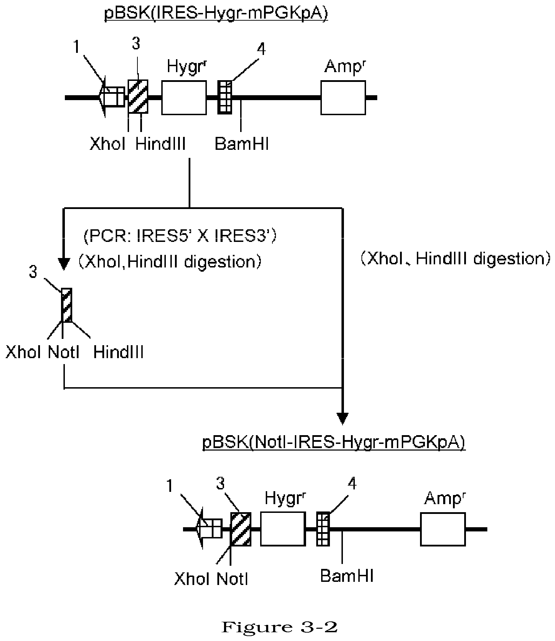

[Fig. 3-2] A diagram illustrating a flow of the method for construction of pE-IRES-GS-puro.

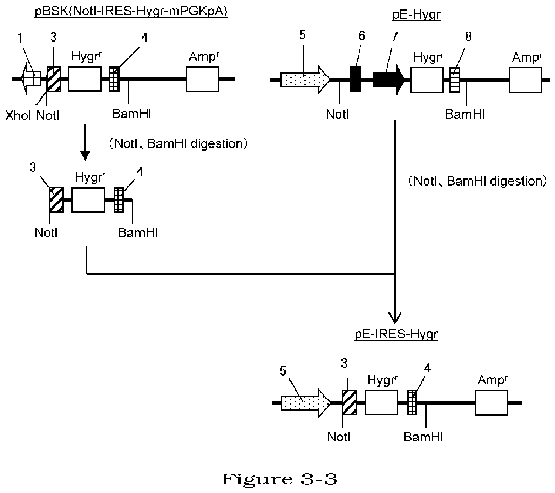

[Fig. 3-3] A diagram illustrating a flow of the method for construction of pE-IRES-GS-puro.
[Fig. 3-4] A diagram illustrating a flow of the method for construction of pE-IRES-GS-puro.
[Fig. 3-5] A diagram illustrating a flow of the method for construction of pE-IRES-GS-puro.
[Fig. 3-6] A diagram illustrating a flow of the method for construction of pE-IRES-GS-puro.
[Fig. 3-7] A diagram illustrating a flow of the method for construction of pE-IRES-GS-puro.
[Fig. 3-8] A diagram illustrating a flow of the method for construction of pE-IRES-GS-puro.
[Fig. 3-9] A diagram illustrating a flow of the method for construction of pE-IRES-GS-puro.

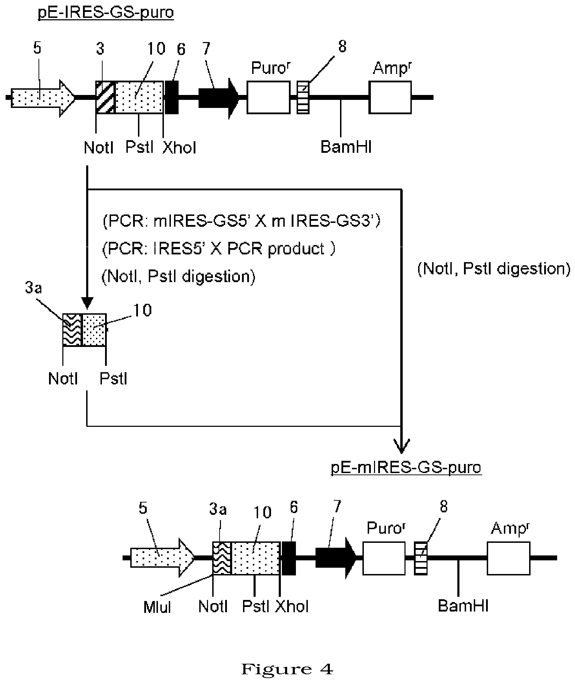

[Fig. 4] A diagram illustrating a flow of the method for construction of pE-mIRES-GS-puro.

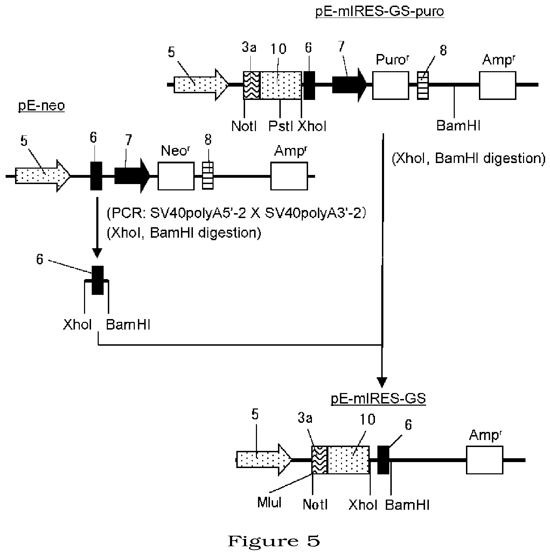

[Fig. 5] A diagram illustrating a flow of the method for construction of pE-mIRES-GS.

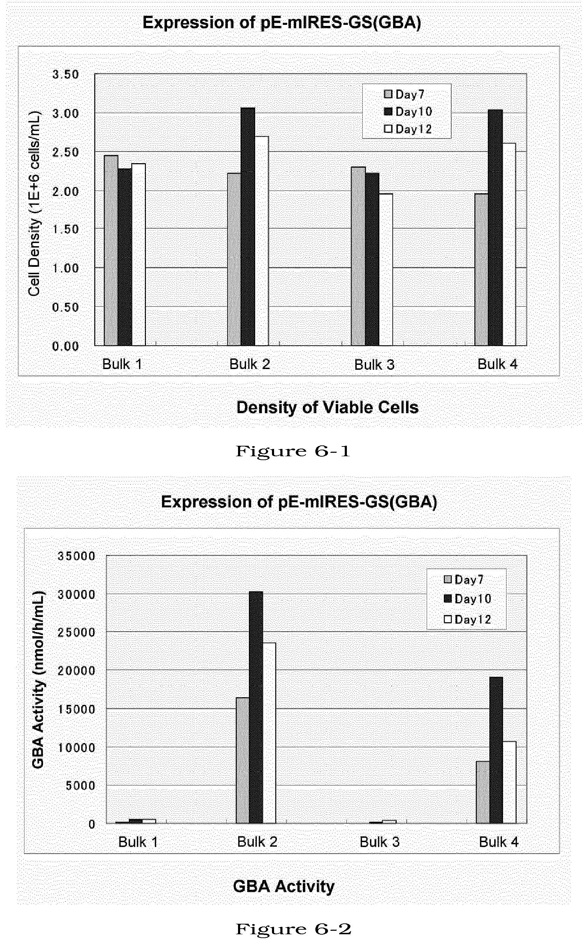

[Fig. 6-1] A figure illustrating viable cell densities of hGBA expressing cells which were cells transformed with an expression vector (pE-mIRES-GS(GBA)).
[Fig. 6-2] A figure illustrating expression levels of glucocerebrosidase (GBA activity) in hGBA expressing cells which were cells transformed with an expression vector (pE-mIRES-GS(GBA)).

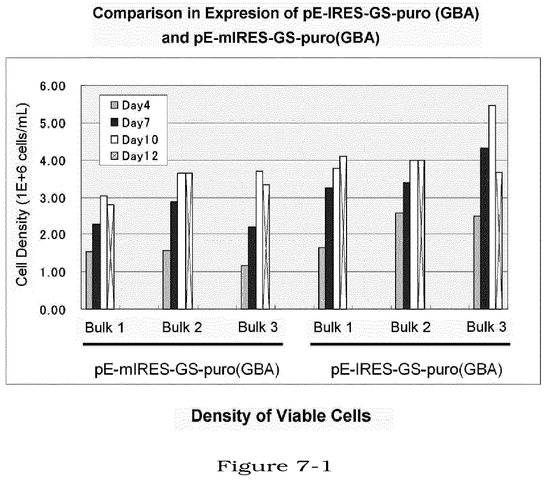

[Fig. 7-1] A figure illustrating viable cell densities of hGBA expressing cells which were cells transformed with an expression vector (pE-IRES-GS-puro(GBA) or pE-mIRES-GS-puro(GBA).

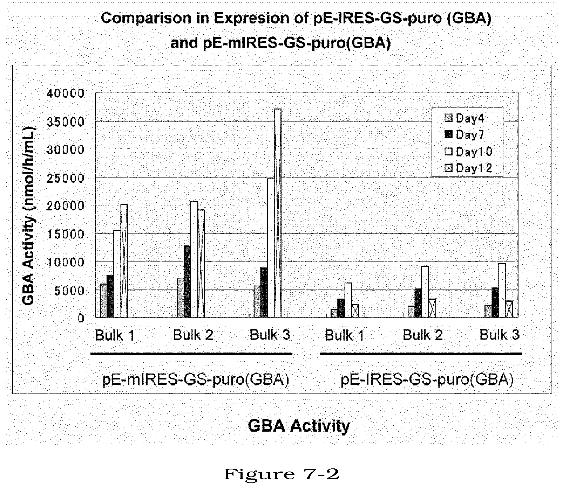

[Fig. 7-2] A figure illustrating expression levels of human glucocerebrosidase (GBA activity) in hGBA expressing cells which were cells transformed with an expression vector (pE-IRES-GS-puro(GBA) or pE-mIRES-GS-puro(GBA)).

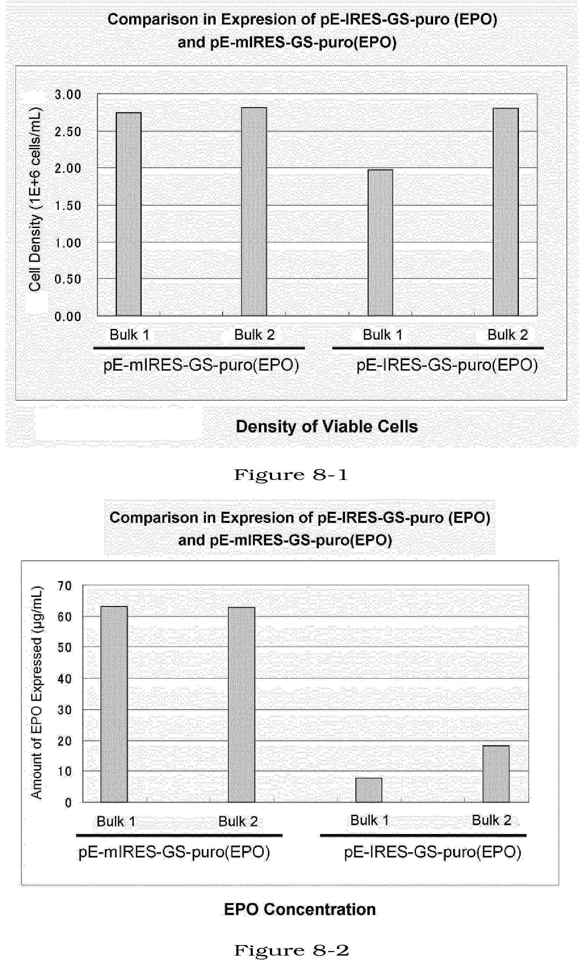

[Fig. 8-1] A figure illustrating viable cell densities of hEPO expressing cells which were cells transformed with an expression vector (pE-IRES-GS-puro(EPO) or pE-mIRES-GS-puro(EPO)).
[Fig. 8-2] A figure illustrating expression levels of human erythropoietin in hEPO expressing cells which were cells transformed with an expression vector (pE-IRES-GS-puro(EPO) or pE-mIRES-GS-puro(EPO)).

### MODE FOR CARRYING OUT THE INVENTION

In the present disclosure the term "gene expression regulatory site" means a DNA region which can regulate the transcription frequency of the gene located downstream thereof, and generally is called a promoter or a promoter gene. A gene expression regulatory site is present upstream of almost every gene which is expressed in the body, regulating the transcription frequency of the gene, and its nucleotide sequence is diverse. Though there is no particular limitation to it as far as it is able to strongly induce expression of a gene incorporated downstream thereof in mammalian cells, a gene expression regulatory site can be a virus-derived promoter, such as a cytomegalovirus (CMV)-derived promoter, SV40 promoter, and the like; and elongation factor 1α (EF-a) promoter, and the like.

In the present disclosure the term "internal ribosome entry site" means a region (structure) inside an mRNA chain to which a ribosome can directly binds and start translation independently from a cap structure, or a region (structure) in a DNA which generates such a region through translation. In the present invention, the term "gene encoding an internal ribosome entry site" means a region (structure) in a DNA which generates such a site through translation. Internal ribosome entry site is generally called IRES, and found in the 5' untranslated region of viruses of *Picornaviridae* (poliovirus, rhinovirus, mouse encephalomyocarditis virus, and the like), *Picornaviridae Aphthovirus,* hepatitis A virus, hepatitis C virus, coronavirus, bovine enterovirus, Theiler's murine encephalomyelitis virus, Coxsackie B virus, and the like, and the 5' untranslated region of human immunoglobulin heavy chain binding protein, drosophila antennapedia gene, drosophila Ultrabithorax gene, and the like. In the case of a picornavirus, its IRES is a region consisting of about 450 bp present in the 5' untranslated region of its mRNA. Here, "5' untranslated region of a virus" means the 5' untranslated region of a viral mRNA, or a region (structure) in a DNA which, when translated, generates such a region.

In the present disclosure there is no particular limitation as to which of internal ribosome entry sites is employed, and any one of them may be used as far as it can act as an internal ribosome entry site in a mammalian cell, in particular a Chinese hamster ovary-derived cell (CHO cell). Among them, preferred is an internal ribosome entry site derived from the 5' untranslated region of a virus, more preferred an internal ribosome entry site derived from the 5' untranslated region of a virus of *Picornaviridae*, and still more preferred an internal ribosome entry site derived from mouse encephalomyocarditis virus.

In the present disclosure internal ribosome entry sites having a wile-type nucleotide sequence may be used directly. Further, any of mutant-type internal ribosome entry sites derived by introducing one or more mutations (such as substitution, deletion, and/or insertion) into one of those wild-type internal ribosome entry site may also be used so long as it can act as an internal ribosome entry site in mammalian cells (especially, CHO cells). Again, a chimeric-type internal ribosome entry site may also be used which is derived by fusion of two or more internal ribosome entry sites.

In addition, in the present invention, placing a gene encoding a glutamine synthetase (GS gene) under the regulation of an internal ribosome entry site, enables control of expression levels of the GS gene. According to such a way of control, if made so that the expression level of the GS gene may fall within a certain range where a sufficient selection pressure works in a selective culture, it is possible to select mammalian cells which express a recombinant protein at high levels, as mentioned later.

Control of the expression level of a GS gene can be achieved by selecting and using as desired such an internal ribosome entry site that brings about enhanced or lowered expression level of the GS gene, from various internal ribosome entry sites It is also possible to achieve this purpose by introducing a mutation into an internal ribosome entry site. In doing this, there is no particular limitation as to the mutation, e.g., the site at which it is introduced, and any mutations may be introduced so long as the expression level of the GS gene present downstream of the internal ribosome entry site is thereby controlled within a certain range as mentioned above.

For example, when introducing a mutation in order to lower the expression level of the GS gene, multiple start codons (ATG) present within a wild-type internal ribosome entry site, each of which could be used as an initiation point of translation, can be a target. For example, destruction of any of such start codons by introduction of a mutation enables lowering of the expression levels of the GS gene incorporated in frame with the start codon. The term "destruction" here means introduction of a mutation into a gene sequence to thereby prevents the intrinsic function of the gene from exhibiting itself. For example, the internal ribosome entry site of the wild-type mouse encephalomyocarditis virus has three start codons (ATG) at its 3' end, whose sequence is set forth as SEQ ID NO:1 (5'-ATGataatATGgccacaaccATG-3': start codons shown in upper letters for clear indication). If it is intended to lower the expression level of the GS gene located downstream of this internal ribosome entry site, the start codon to be destroyed by introduction of a mutation into it is preferably the 2nd or 3rd start codon from the 5' end, more preferably the 2nd start codon. Thus, examples of an internal ribosome entry sites containing such an introduced mutation includes those having at their 3' end a nucleotide sequence set forth as SEQ ID NO:2 (5'-atgataatnnngccacaaccnnn-3' : n representing any nucleotide), or a nucleotide sequence set forth as SEQ ID NO:32 (5'-atgataannnngccacaaccnnn-3': n representing any nucleotide), or a nucleotide sequence set forth as SEQ ID NO:3 (5'-atgataatnnngccacaaccatg-3': n representing any nucleotide), or a nucleotide sequence set forth as SEQ ID NO:33 (5'-atgataannnngccacaaccatg-3': n representing any nucleotide). More specifically, an internal ribosome entry site having at its 3' end a nucleotide sequence set forth as SEQ ID NO:4 (5'-atgataagcttgccacaaccatg-3'), in which the 2nd start codon from the 5' end has been destroyed by mutation.

Still more specifically, the internal ribosome entry site of the wild-type mouse encephalomyocarditis virus comprises a nucleotide sequence set forth as SEQ ID NO:5

Further, an example of nucleotide sequences which is derived by introducing a mutation into the above nucleotide sequence is the one set forth as SEQ ID NO:6

Furthermore, the expression levels of a GS gene located downstream of a wild-type and/or a mutant-type internal ribosome entry site may be controlled by other methods. For example, when it is intended to lower the expression level of a GS gene, lowered expression level of the gene can also be achieved either by incorporating the GS gene in an out-of-frame fashion of the start codon in the internal ribosome entry site or by introducing a nucleotide sequence that inhibits transcription or translation between the internal ribosome entry site and the GS gene located downstream thereof. There is no particular limitation as to a nucleotide which inhibits transcription, so long as it inhibits transcription of the GS gene incorporated downstream of the internal ribosome entry site. Examples include the polymerase addition signal (5'-aataaa-3') and the like. Examples of such nucleotide sequences that inhibits translation include those inhibit proper translation, such as a stop codon that induces a reading through, though there is no particular limitation so long as they inhibit translation of the gene incorporated downstream of the internal ribosome entry site.

In the present invention, there is no particular limitation as to the term "glutamine synthetase" so long as it can synthesize glutamine from glutamic acid and ammonia, and it may be of any origin including mammals, reptiles, birds, amphibians, insects such as *Bombyx mori, Spodoptera frugiperda, Geometridae,* and the like, of *Lepidoptera*; *Drosophila* of *Diptera*; procaryotes; nematodes; yeasts; actinomycetes; filamentous fungi; ascomycetes; Basidiomycota; and plants. Among these, preferred are those originating from mammals, and one originating from human or Chinese hamster (esp. originating from Chinese hamster) may be preferably used.

Furthermore, there is no particular limitation as to the term "glutamine synthesis inhibiter", and any compound may be used so long as it can inhibit the activity of the glutamine synthetase mentioned above. Preferred examples include methionine sulfoximine (MSX)

In the present disclosure an expression vector may comprise an additional selection marker introduced to it in addition to a GS gene. Such an additional selection marker is a gene which can give drug resistance to the mammalian cells into which the expression vector has been introduced (drug resistance gene). In the present invention, there is no particular limitation as to genes which can be used as drug resistance genes, so far as they can provide mammalian cells with drug resistance. But preferred are genes which can provide cells with resistance to such drugs as puromycin, hygromycin, blasticidin, neomycin, and the like. With this regard, drugs such as puromycin, hygromycin, blasticidin, neomycin, and the like are "drugs corresponding to the drug resistance genes", respectively. Among these drug resistance genes, more preferred examples include a puromycin resistance gene, a hygromycin resistance gene, a blasticidin resistance gene, and a neomycin resistance gene.

In the present disclosure expression levels of a drug resistance gene may be regulated by incorporating it downstream of a separate gene expression regulatory site (second gene expression regulatory site) provided separately from the gene expression regulatory site by which a recombinant protein is regulated. In this case, such a second gene-regulatory site is employed that allows control of the expression level of the drug resistance gene to fall in a region in which it provides sufficient selection pressure in a selective culture. Namely, by relatively suppressing the expression level of a drug resistance gene, it is possible to increase the drug sensitivity of the mammalian cells transformed with the expression vector, thereby to induce a higher level expression of the gene encoding a protein of interest, thus enabling selection of those mammalian cells which express the recombinant protein at high levels.

Further, in the present disclosure a drug resistance gene may, accompanied by a second internal ribosome entry site upstream thereof, be incorporated, either in a region between the gene encoding a recombinant protein and the internal ribosome entry site, or in a region downstream of the GS gene. By this, the expression level of the drug resistance gene can be controlled with the second internal ribosome entry site. In this case, the second internal ribosome entry site employed may be either the same as the internal ribosome entry site upstream of the GS gene or a different one. Further, a second ribosome entry site may be selected as desired from the various internal ribosome entry sites mentioned above. As regards a second internal ribosome entry site, it is also possible to control the expression level of the drug resistance gene by selecting a proper one or introducing a mutation into it.

In the present disclosure there is no particular limitation as to the species of an animal whose gene is incorporated, as encoding an recombinant protein, into an expression vector, whether or not it originates from mammal including human. For example, such a gene is generally of human origin if the expression vector according to the present invention is used for production of ethical pharmaceuticals, and generally originating from a domestic animal to be treated if the expression vector is used for production of drugs for domestic animals. Again, there is no particular limitation as to which protein of interest a gene encodes, either, but preferred are such genes that encode lysosomal enzymes including α-galactosidase A, iduronate-2-sulfatase, glucocerebrosidase, galsulfase, α-L-iduronidase, and acid α-glucosidase; tissue plasminogen activator (t-PA); blood coagulation factors including blood coagulation factor VII, blood coagulation factor VIII, and blood coagulation factor IX; erythropoietin, interferons, thrombomodulin, follicle stimulating hormone, granulocyte colony-stimulating factor (G-CSF); or various antibody medicaments. Among these, more preferred are genes encoding lysosomal enzymes and erythropoietin, and still more preferred are genes encoding glucocerebrosidase and erythropoietin.

In the present disclosure, there is no particular limitation as to mammalian cells into which an expression vector according to the present invention is introduced, so long as they can express an aimed recombinant protein, and they may be primary culture of the cells collected from organs, muscle tissues, skin tissues, connective tissue, nerve tissue, blood, bone marrow, and the like taken out of the body, or their secondary culture cells or cell lines established so as to keep their characteristics through repeated subcultures. Those cells may be either normal cells or cells which have become cancerous. Cells which can be used particularly preferably are CHO cells, which are derived from the ovary of a Chinese hamster; human fibroblasts; and COS cells, which are derived from the renal fibroblast of an African green monkey.

In the present disclosure, introduction of an expression vector into mammalian cells is made for the purpose of letting a gene encoding a recombinant protein express itself in the mammalian cells. Thus, it may be made by any methods so long as the they meet their purpose. An expression vector is a circular plasmid in general, and it may be introduced into cells either in that circular form or after cleaved with a restriction enzyme to make it linear.

Mammalian cells into which an expression vector has been introduced (expression vector-introduced cells) then are cultured in a glutamine-free, or low glutamine medium, containing a glutamine synthetase inhibitor (e.g, MSX), (and further containing a drug corresponding to a drug resistance gene, e.g., an antibiotic or the like, where applicable), and only those cells are selected which express the GS gene (so-called a selection marker)(and further the drug resistance gene, where applicable) in them. This is referred to as a selective culture, and the medium used here a selective medium.

In a selective culture, if the selection marker is expressed too much relative to the amount of the GS inhibitor or the drug, an insufficient selection pressure will result and thus no expression vector-introduced cells can be obtained which express relatively higher levels of the recombinant protein, whereas if the selection marker is expressed all too little, no expression vector-introduced cells with relatively higher expression levels can be obtained, either, because of death or insufficient growth of the cells. In contrast, if the expression level of the selection marker is adjusted in a certain range so that increased sensitivity to the GS inhibitor or the drug and sufficient exposure to selection pressure are available, expression vector-introduced cells can be obtained which express the recombinant protein at relatively higher levels, as mentioned below.

Namely, adjustment of the level of expression of the selection marker in a certain range so that a sufficient selection pressure is available in a selective culture, followed by a stepwise increase of the concentration of a GS inhibitor (and further the concentration of the drug corresponding to the drug resistance marker, where applicable) added to the selective medium, will allow one to select expression vector-introduced cells which express the selection marker at higher levels. This is partly due to the fact that the copy number of the selection marker incorporated in the genome of the expression vector-introduced cells multiplies in the process of the selective culture, and among the expression vector-introduced cells, only those with elevated expression levels of the selection marker thus will selectively grow. As the copy number of the gene encoding the recombinant protein incorporated in the expression vector also increases at the same time, the expression levels of the gene also increases. Thus, expression vector-introduced cells with relatively higher expression levels of the recombinant protein of interest can be selected by in this manner of selective culture of the expression vector-introduced cells. In the present specification, expression vector-introduced cells thus selected is referred to as transformed cells.

In the present disclosure, if an expression vector in which a drug resistance gene in addition to the GS gene are incorporated as selection markers are introduced into mammalian cells, transformed cells with further increased expression levels can be obtained as compared with the case where the GS gene alone is incorporated.

In the present disclosure, where the concentration of a GS inhibitor or a drug corresponding to the drug resistance gene added to a selective medium is increased stepwise, their maximum concentration is preferably 100-1000 µM, more preferably 200-500 µM, and still more preferably about 300 µM where the GS inhibitor is methionine sulfoximine, for example. Where the drug is puromycin, its maximum concentration is preferably 3-30 µM, more preferably 5-20 µM, and still more preferably about 10 µM.

### EXAMPLES

Though the present invention will be described in further detail below with reference to examples, it is not intended that the present invention be limited to the examples.

### [Construction of pE-neo vector and pE-hygr vector]

pEF/myc/nuc vector (Invitrogen) was digested with KpnI and NcoI to cut out a region which includes EF-1 promoter and its first intron, which then was blunt-ended with T4 DNA polymerase. pCl-neo (Invitrogen), after digested with BglII and EcoRI to remove a region containing CMV enhancer/promoter and introns, was blunt-ended with T4 DNA polymerase. Into this was inserted the above-mentioned region including EF-1α promoter and its first intron to construct pE-neo vector (Fig. 1-1 and Fig. 1-2).

pE-neo vector was digested with SfiI and BstXI to cut out a region of about 1 kbp including a neomycin resistance gene (Fig. 2-1). A hygromycin resistance gene was amplified by PCR using pcDNA3.1/Hygro(+) (Invitrogen), as a template, and primer Hyg-Sfi5' (5'-gaggccgcctcggcctctga-3'; SEQ ID NO:7) and primer Hyg-BstX3' (5'-aaccatcgtgatgggtgctattcctttgc-3'; SEQ ID NO:8)(Fig. 2-2). The hygromycin gene thus amplified then was digested with SfiI and BstXI and inserted into pE-neo vector mentioned above to construct pE-hygr vector (Fig. 2-3).

### [Construction of pE-IRES-GS-puro]

An expression vector pPGKIH (Miyahara M. et.al., J. Biol. Chem. 275,613-618(2000)) was digested with restriction enzymes (XhoI and BamHI) to cut out a DNA fragment consisting of a following nucleotide sequence IRES-Hygr-mPGKpA, which included an internal ribosome entry site (IRES) derived from mouse encephalomyocarditis virus (EMCV), a hygromycin resistance gene (Hygr gene), and the polyadenylation region (mPGKpA) of mouse phosphoglycerate kinase (mPGK): the sequence written in capital letters ,"CTCGAG", at the 5'-end represents a "XhoI site"; the next sequence written in capital letters starting with "CGC" and the region consisting of three small letters (atg) which follows together represents a "nucleotide sequence including the internal ribosome entry site derived from the 5' untranslated region of mouse encephalomyocarditis virus"; the region written in small letters starting with the atg represents the "nucleotide sequence encoding a hygromycin resistance gene"; the region which follows and starts with small letters "aaa" represents a "nucleotide sequence including the polyadenylation region of mouse phosphoglycerate kinase"; and the sequence written in capital letters "GGATCC" at the 3' end represents a "BamHI site"). (Besides, the amino acid sequence corresponding to the Hygr gene is set forth as SEQ ID NO:10). This DNA fragment then was inserted into pBluescript SK(-)(Stratagene) between its XhoI and BamHI sites, and the resulting product was designated pBSK(IRES-Hygr-mPGKpA) (Fig. 3-1).

A DNA fragment containing part of the IRES of EMCV was amplified by PCR using pBSK (IRES-Hygr-mPGKpA), as a template, and primer IRES5' (5'-caactcgagcggccgccccccccccctctccctcccccccccctaacgttact-3'; SEQ ID NO:11) and primer IRES3' (5'-caagaagcttccagaggaactg-3'; SEQ ID NO:12). This fragment then was digested with restriction enzymes (XhoI and HindIII) and inserted into pBSK(IRES-Hygr-mPGKpA) between its XhoI and HindIII sites, and the resulting product was designated pBSK(NotI-IRES-Hygr-mPGKpA) (Fig. 3-2).

pBSK(NotI-IRES-Hygro-mPGKpA) was digested with restriction enzymes (NotI and BamHI) and inserted into pE-hygr vector between its NotI and BamHI sites, and the resulting product was designated plasmid pE-IRES-Hygr (Fig. 3-3).

Using the expression vector pPGKIH, as a template, and primer mPGKP5' (5'-gcgagatcttaccgggtaggggaggcgctt-3'; SEQ ID NO:13) and primer mPGKP3' (5'-gaggaattcgatgatcggtcgaaaggcccg-3'; SEQ ID NO:14), PCR was performed to amplify a DNA fragment consisting of a following nucleotide sequence including the promoter region of mPGK (mPGKp): NO:15, the first sequence from the 5' end written in small letters "agatct" represents a "BglII site", the sequence written in capital letters starting with "TAC" which follows represents a "nucleotide sequence including the promoter region of mouse phosphoglycerate kinase", and the sequence written in capital letters "GAATTC" which follow represents an "EcoRI site"). This DNA fragment then was digested with restriction enzymes (BglII and EcoRI) and inserted into pCI-neo (Promega) into its BglII and EcoRI sites, and the resulting product was designated pPGK-neo (Fig. 3-4).

pE-IRES-Hygr was digested with restriction enzymes (NotI and BamHI) to cut out a DNA fragment (IRES-Hygr), and this was inserted into pPGK-neo between its NotI and BamHI sites. The resulting product was designated pPGK-IRES-Hygr (Fig. 3-5).

cDNA was prepared from CHO-K1 cells, and using it, as a template, and primer GS5' (5'-aatatggccacaaccatggcgacctcagcaagttcc-3'; SEQ ID NO:16) and primer GS3' (5'-ggaggatccctcgagttagtttttgtattggaagggct-3'; SEQ ID NO:17), PCR was performed to amplify a DNA fragment including the GS gene. The DNA fragment was digested with restriction enzymes (BalI and BamHI) and inserted into pPGK-IRES-Hygr between its Ball and BamHI sites. The resulting product was designated pPGK-IRES-GS-ΔpolyA (Fig. 3-6).

Using pCAGIPuro (Miyahara M. et.al., J. Biol. Chem. 275,613-618(2000)), as a template, and primer puro5' (5'-gcttaagatgaccgagtacaagcccacg-3'; SEQ ID NO:18) and primer puro3' (5'-cccatcgtgatggtcaggcaccgggcttgc-3'; SEQ ID NO:19), PCR was performed to amplify a following nucleotide sequence including a puromycin resistance gene (puro gene): (5'-GcttaagATGACCGAGTACAAGCCCACGGTGCGCCTCGCCACCCGCGACGACGTCCCCA GGGCCGTACGCACCCTCGCCGCCGCGTTCGCCGACTACCCCGCCACGCGCCACACCGTC GATCCGGACCGCCACATCGAGCGGGTCACCGAGCTGCAAGAACTCTTCCTCACGCGCGTC GGGCTCGACATCGGCAAGGTGTGGGTCGCGGACGACGGCGCCGCGGTGGCGGTCTGGA CCACGCCGGAGAGCGTCGAAGCGGGGGCGGTGTTCGCCGAGATCGGCCCGCGCATGGC CGAGTTGAGCGGTTCCCGGCTGGCCGCGCAGCAACAGATGGAAGGCCTCCTGGCGCCGC ACCGGCCCAAGGAGCCCGCGTGGTTCCTGGCCACCGTCGGCGTCTCGCCCGACCACCAG GGCAAGGGTCTGGGCAGCGCCGTCGTGCTCCCCGGAGTGGAGGCGGCCGAGCGCGCCG GGGTGCCCGCCTTCCTGGAGACCTCCGCGCCCCGCAACCTCCCCTTCTACGAGCGGCTC GGCTTCACCGTCACCGCCGACGTCGAGGTGCCCGAAGGACCGCGCACCTGGTGCATGAC CCGCAAGCCCGGTGCCTGAccatcacgatggG-3'; SEQ ID NO:20, the first sequence from the 5' end written in small letters "cttaag" represents a "AflII" site, the sequence written in capital letters and starting with "ATG " which follows represents "sequence encoding the puromycin resistance gene (puro gene)", and the sequence written in small letters which follow represents a "BstXI site") (Besides, the amino acid sequence corresponding to the puro gene is set forth as SEQ ID NO:21). The DNA fragment was digested with restriction enzymes (AflII and BstXI) and inserted into the expression vector pE-neo between its AflII and BstXI sites. The resulting product was designated pE-puro (Fig. 3-7).

Using pE-puro, as a template, and primer SV40polyA5' (5'-caacaagcggccgccctcgagttccctttagtgagggttaatgc-3'; SEQ ID NO:22) and primer SV40polyA3' (5'-cccctgaacctgaaacataaaatg-3'; SEQ ID NO:23), PCR was performed to amplify a DNA fragment including SV40 late polyadenylation region. The DNA fragment then was digested with restriction enzymes (NotI and HpaI) and inserted into pE-puro between its NotI and HpaI sites. The resulting product was designated pE-puro(XhoI) (Fig 3-8).

pPGK-IRES-GS-ΔpolyA was digested with restriction enzymes (NotI and XhoI) to cut out a DNA fragment including the IRES-GS region, which then was inserted into the expression vector pE-puro(XhoI) between its NotI and XhoI sites. The resulting product was designated pE-IRES-GS-puro (Fig. 3-9).

### [Construction of pE-mIRES-GS-puro]

Using the expression vector pE-IRES-GS-puro, as a template, and primer mIRES-GS5' (5'-acacgatgataagcttgccacaacc-3'; SEQ ID NO:24) and primer mIRES-GS3' (5'-ctccacgatatccctgccata-3'; SEQ ID NO:25), PCR was performed to amplify a region from the IRES to GS of EMCV, and thus a DNA fragment was amplified in which the second start codon (ATG) from the 5' end of the IRES of EMCV was broken by introduction of a mutation. Using the expression vector pE-IRES-GS-puro, as a template, and the DNA fragment and the above-mentioned primer IRES5', PCR was performed to amplify a DNA fragment including a region from IRES to GS. This DNA fragment was digested with restriction enzymes (NotI and PstI), and a DNA fragment thus cut out was inserted into the expression vector pE-IRES-GS-puro between its NotI and PstI sites. The resulting product was designated pE-mIRES-GS-puro (Fig. 4).

### [Construction of pE-mIRES-GS]

Using the expression vector pE-neo, as a template, and primer SV40polyA5'-2 (5'-actaactcgagttccctttagtg-3'; SEQ ID NO:26) and primer SV40polyA3'-2 (5'-aacggatccttatcggattttaccac-3'; SEQ ID NO:27), PCR was performed to amplify a DNA fragment including the SV40 polyA region. This DNA fragment was digested with restriction enzymes (XhoI and BamHI) and inserted into pE-mIRES-GS-puro between its XhoI and BamHI sites. The resulting product was designated pE-mIRES-GS (Fig. 5).

### [Construction of human glucocerebrosidase (hGBA) expression vector]

Using a human liver Quick Clone cDNA (Clontech), as a template, and primer hGBA5' (5'-gcaatacgcgtccgccaccatggagttttcaagtccttccagagagg-3'; SEQ ID NO:28) and primer hGBA3' (5'-ggacgcggccgcgagctctcactggcgacgccacaggtagg-3'; SEQ ID NO:29), PCR was performed to amplify a DNA fragment including the human glucocerebrosidase gene (hGBA gene). This DNA fragment was digested with restriction enzymes (MluI and NotI) and inserted into pE-IRES-GS-puro, pE-mIRES-GS, and pE-mIRES-GS-puro, respectively, between their MluI and NotI sites to provide GBA expression vectors, pE-IRES-GS-puro(GBA), pE-mIRES-GS(GBA), and pE-mIRES-GS-puro(GBA), respectively.

### [Construction of human erythropoietin (hEPO) expression vector]

Using pCI-neo(EPO), as a template, primer hEPO5' (5'-aagacgcgtcgccaccatgggggtgcacgaatgtcctgc-3'; SEQ ID NO:30), and primer hEPO3' (5'-aagagcggccgctcatctgtcccctgtcctgcagg-3'; SEQ ID NO:31), PCR was performed to amplify a DNA fragment including the hEPO gene. This DNA fragment was digested with restriction enzymes (MluI and NotI) and inserted into pE-IRES-GS-puro and pE-mIRES-GS-puro between their MluI and NotI sites to provide hEPO expression vectors, pE-IRES-GS-puro(EPO) and pE-mIRES-GS-puro(EPO), respectively.

### [Preparation of hGBA expressing cells and hEPO expressing cells]

Into CHO-K1 cells, which were cells derived from the ovary of a Chinese hamster, were introduced pE-IRES-GS-puro(GBA), pE-mIRES-GS(GBA), pE-mIRES-GS-puro(GBA), pE-IRES-GS-puro(EPO), and pE-mIRES-GS-puro(EPO), respectively, using Lipofectamine 2000 reagent (Invitrogen). The resulting cells then were subjected to selective culture in selective media to provide hGBA expressing transformant cells and hEPO expressing transformant cells.

In this, a CD Opti CHO medium (Invitrogen) containing methionine sulfoximine (SIGMA) and puromycin (SIGMA) was used as the selective medium for the selective culture of the cells into which pE-IRES-GS-puro(GBA), pE-mIRES-GS-puro(GBA), pE-IRES-GS-puro(EPO), or pE-mIRES-GS-puro(EPO) had been introduced, and a CD Opti CHO medium (Invitrogen) containing methionine sulfoximine (SIGMA) was used as a selective medium for the selective culture of the cells into which pE-mIRES-GS(GBA) had been introduced. During the selective culture, the concentration of methionine sulfoximine and puromycin was increased stepwise up to the final concentration of 300 µM for methionine sulfoximine and 10 µg/mL for puromycin to let those cells exhibiting drug resistance grow selectively. By this selective culture, three-types of hGBA expressing transformant cells and two-types of hEPO expressing transformant cells were obtained.

### [Culture of hGBA expressing cells and measurement of cell density]

Those transformant cells obtained after the selective culture then were cultured, at their cell density of 2 × 10⁵ cells/mL, in 5 mL of a CD Opti CHO medium containing 300 µM methionine sulfoximine and 10 µg/mL puromycin, for 12 days under 5 % CO₂. The temperature in this culture was set at 37° C from the start to day 3 of the culture, and at 30° C thereafter. The supernatant of the culture was sampled on days 4, 7, 10, and 12 to measure its cell density. Besides, the culture of the hGBA expressing transformant cells obtained by introduction of pE-mIRES-GS(GBA) was carried out in 5 mL of Opti CHO medium containing 300 µM methionine sulfoximine.

### [Culture of hEPO expressing cells and measurement of cell density]

Those transformant cells obtained after the selective culture then were cultured, at their cell density of 2 × 10⁵ cells/mL, in 5 mL of a CD Opti CHO medium containing 300 µM methionine sulfoximine and 10 µg/mL puromycin, for 7 days under 5 % CO₂. The temperature in this culture was set at 37° C from the start to day 3 of the culture, and at 30° C thereafter. The supernatant of the culture was sampled on day 7 of the culture to measure its cell density.

### [Measurement of hGBA activity]

Measurement of GBA activity was performed in accordance with the method described in Pasmanik-Chor M. et al., Biochem J 317, 81-88 (1996). Namely, 4-methylumbelliferyl phosphate (4-MUF, Sigma Chemical Co.) was dissolved in a dilution buffer (100 mM potassium phosphate buffer (pH 5.96) containing 0.125 % sodium taurocholate, 0.15% Triton X-100, and 0.1 % bovine serum albumin) and diluted stepwise to prepare standard solutions with their concentration adjusted to 200, 100, 50, 25, 12.5, 6.25, and 3.125 mM. 4-methylumbelliferyl-β-D-glucopyranoside (Sigma Chemical Co.) was dissolved in the dilution buffer at a concentration of 4 mM, and the resulting solution was used as the substrate solution. Samples were diluted with the dilution buffer, where needed, before measurement. The 4-MUF standard solutions or samples were added, 10 µL each, to a FluoroPlate F96, and then 70 µL each of the substrate solution was admixed. After a one-hour reaction at 37° C, 200 µL of 50 mM glycine-NaOH buffer (pH 10.6) was added to each well as a reaction terminator solution, and fluorescence intensity was measured on a FluoroPlate reader under a condition of excitation wavelength of 355 nm and detection wavelength of 460 nm. A standard curve was produced based on the fluorescence intensity of the 4-MUF standard solutions, and the fluorescence intensities of the samples were interpolated on it to calculate its activity (nmol/h/mL).

### [Quantitative determination of hEPO by ELISA]

A solution of rabbit anti-hEPO antibody was prepared in a conventional manner from the blood of a rabbit immunized with a recombinant hEPO. This recombinant hEPO had been prepared with reference to the method described in a published international application (WO 2008/068879). This rabbit anti-hEPO antibody solution was added, 100 µL each, to a 96-well plate and was allowed to stand for one hour at 4° C to let antibody adhere to the plate. After the solution was discarded, 1 % BSA/TBS-T solution (Tris: 0.005 M, NaCl: 0.138 M, KCl: 0.0027 M, pH 8.0) containing 0.075 % Tween 20 was added, 100 µL each, to the plate, and the plate then was let stand for one hour at 4° C to block the plate. The solution was discarded, and the plate was washed three times with a TBS-T solution containing 0.075 % Tween 20. Then, samples diluted to proper concentrations were added, 100 µL each, to the plate, and the plate was let stand for one hour at 37° C. In parallel, the homemade hEPO, whose quantity had been determined by the Lawry method, was diluted to concentrationss of 1-16 ng/mL, and the resulting solutions were added, as standard solutions, to the plate in the same manner as the samples, and the plate was let stand. The solution was discarded, and after the plate was washed as described above, HRP-labeled mouse anti hEPO monoclonal antibody (mfd by R&D) was added, 100 µL each, to the plate as a secondary antibody, and the plate was let stand for one hour at 37° C. The plate, after washing as described above and addition of HRP substrate (Promega), was let stand for 15 minutes at 37° C, and hydrochloric acid was added to terminate the reaction. Absorbance at 450 nm was measured on a Microwell Plate Reader, and the hEPO concentration in each sample was determined from comparison with the standard solutions in their absorbance.

### [Results]

As described above, the CHO cells carrying the introduced pE-mIRES-GS (GBA), the expression vector in which were incorporated the elongation factor 1α promoter (EF-1p) as a gene expression regulatory site, the human glucocerebrosidase (hGBA) gene as a gene encoding a protein, the internal ribosome entry site (EMCV-mIRES) including the nucleotide sequence set forth as SEQ ID NO:4 derived from a mutant-type mouse encephalomyocarditis virus as an internal ribosome entry site, and a gene encoding a glutamine synthetase (GS gene) in this order, were cultured in a selective medium, and hGBA activity of the medium was measured and the cell density as well. The experiment was carried out four times (bulks 1-4) separately. The cell density nearly reached 2.5 × 10⁶ cells/mL on day 7 of the culture in each of the four runs (Fig. 6-1). On the other hand, the hGBA activity in the medium reached 30 µmol/h/mL on day 10 of the culture with bulk 2 (Fig. 6-2), confirming that transformation of CHO cells with pE-mIRES-GS(GBA) enables production of cells which express hGBA at high levels. However, the hGBA activity in the medium was very low with bulks 1 and 3, which suggested that with pE-mIRES-GS(GBA), cells after a selective culture included at significant proportions of those cells expressing only a low level of hGBA.

Then, the CHO cells carrying the introduced pE-IRES-GS-puro(GBA), the expression vector in which were incorporated EF-1p as a gene expression regulatory site, the hGBA gene as a gene encoding a protein, the internal ribosome entry site (EMCV-IRES) including the nucleotide sequence set forth as SEQ ID NO:1 derived from a wild-type mouse encephalomyocarditis virus as an internal ribosome entry site, and the GS gene in this order, and was further incorporated a puromycin gene (puro gene) as a drug resistance gene, were cultured in a selective medium, and the hBGA activity of the medium was measured and the cell density as well. The experiment was carried out three times (bulks 1-3) separately. The cell density about reached 3-4 × 10⁶ cells/mL on day 7 of the culture in each of the three runs, and, especially, exceeded 4 × 10⁶ cells/mL in bulk 3 (Fig. 7-1, right). On the other hand, the hGBA activity in the medium reached 5-10 µmol/h/mL on day 10 of the culture in all of the three runs (Fig. 7-2, right), confirming that transformation of CHO cells with pE-IRES-GS-puro(GBA) enables production of cells which express hGBA at high levels.

Then, the CHO cells carrying the introduced pE-mIRES-GS-puro(GBA), the expression vector in which were incorporated EF-1p as a gene expression regulatory site, hGBA gene as a gene encoding a protein, EMCV-mIRES as an internal ribosome entry site, and a GE gene in this order, and was further incorporated a puro gene as a drug resistance gene, were cultured in a selective medium, and the hGBA activity of the medium was measured and the cell density as well. The experiment was carried out three times (bulks 1-3) separately. The cell density nearly reached 2-3 × 10⁶ cells/mL on day 7 of the culture in all the three runs (Fig. 7-1, left). On the other hand, the hGBA activity of the medium reached 15-25 µmol/h/mL on day 10 in all the three runs (Fig. 7-2, left), and its expression levels thus was remarkably higher even than the expression levels of hGBA in the CHO cells transformed with pE-IRES-GS-puro(GBA), confirming that transformation of CHO cells with pE-mIRES-GS-puro(GBA) enables production of cells which express hGBA at very high levels. In particular, with bulk 3, the hBGA activity exceeded 35 µmol/h/mL on day 12 of the culture, thus exhibiting excellently high levels of hGBA expression.

Then, the CHO cells carrying the introduced pE-IRES-GS-puro(EPO), the expression vector in which were incorporated EF-1p as a gene expression regulatory site, the human erythropoietin gene (hEPO gene) as a gene encoding a protein, EMCV-IRES as an internal ribosome entry site, and the GS gene in this order, and further was incorporated the puro gene as a drug resistance gene, were cultured in a selective medium, and concentration of the hEPO in the medium was measured and the cell density as well.

The experiment was carried out two times (bulks 1-2) separately. The cell density nearly reached 2-3 × 10⁶ cells/mL on day 7 of the culture in both the two runs (Fig. 8-1, right). On the other hand, the hEPO concentration in the medium reached 8-18 µg/mL on day 7 of the culture (Fig. 8-2, right), confirming that transformation of CHO cells with pE-IRES-GS-puro(EPO) enables production of cells which express hEPO at high levels.

Then, the mammalian cells carrying the introduced pE-mIRES-GS-puro(EPO), the expression vector in which were incorporated EF-1p as a gene expression regulatory site, hEPO gene as a gene encoding a protein, EMCV-mIRES as an internal ribosome entry site, and the GE gene in this order, and further was incorporated the puro gene as a drug resistance gene, were cultured in a selective medium, and the concentration of the hEPO was measured and the cell density as well. The experiment was carried out two times (bulks 1-2) separately. The cell density nearly reached 2.5-3 × 10⁶ cells/mL on day 7 in both of the runs (Fig. 8-1, left). On the other hand, the hEPO activity in the medium reached about 63 µg/mL on day 7 of the culture in both of the two runs (Fig. 8-2, left). The expression levels thus was remarkably higher even than the expression levels of hGBA in the CHO cells transformed with pE-IRES-GS-puro(EPO), confirming that transformation of CHO cells with pE-mIRES-GS-puro(EPO) enables production of cells which express hEPO at excellently high levels.

The above results indicate that either an expression vector which contains, downstream of a gene expression regulatory site, a gene encoding a protein of interest, an internal ribosome entry site having a nucleotide sequence set forth as SEQ ID NO: 1 or SEQ ID NO:4, and glutamine synthetase, in this order, or an expression vector which contains, downstream of a gene expression regulatory site, a gene encoding a protein of interest, an internal ribosome entry site having a nucleotide sequence set forth as SEQ ID NO: 1 or SEQ ID NO:4, a glutamine synthetase, in this order, and a drug resistance gene in addition, is a vector which can express the gene encoding the protein of interest at high levels. In particular, the results indicate that an expression vector containing, downstream of an elongation factor 1α promoter, a gene encoding a protein, an internal ribosome entry site having a nucleotide sequence set forth as SEQ ID NO:4, and a glutamine synthetase, in this order, and a puromycin resistance gene in addition as a drug resistance gene, is an expression vector which can express the gene encoding the protein at high levels.

### INDUSTRIAL APPLICABILITY

As the present invention enables expression of a recombinant protein at high levels using mammalian cells, it can realize, for example, a great reduction of production cost of ethical drugs containing a recombinant protein.

### DESCRIPTION OF SIGNS

1 LacZ promoter
2 mPGK promoter
3 Internal ribosome entry site (EMCV-IRES) derived from wild-type mouse encephalomyocarditis virus, containing a nucleotide sequence set forth as SEQ ID NO:1
3a Internal ribosome entry site (EMCV-mIRES) derived from mutant-type mouse encephalomyocarditis virus, containing a nucleotide sequence set forth as SEQ ID NO:4
4 Polyadenylation region (mPGKpA) of mPGK
5 Nucleotide sequence containing EF-1p and the first intron
6 SV40 late polyadenylation region
7 SV40 early promoter region
8 Synthetic polyadenylation region
9 Region containing cytomegalovirus promoter
10 Glutamine synthetase gene

### [SEQUENCE LISTING]

### GP151-PCT ST25.txt

## Claims

1. An expression vector for expression of a protein, comprising a gene expression regulatory site that is an elongation factor 1α promoter, a gene encoding the protein downstream thereof, an internal ribosome entry site derived from the 5' untranslated region of mouse encephalomyocarditis virus and comprising the nucleotide sequence set forth as SEQ ID NO: 4 or SEQ ID NO: 6, further downstream thereof, a gene encoding a glutamine synthetase still further downstream thereof, and an additional gene expression regulatory site and a puromycin resistance gene downstream thereof.

2. The expression vector according to claim 1, wherein the additional gene expression regulatory site is selected from the group consisting of cytomegalovirus derived promoter, SV40 early promoter, and elongation factor 1α promoter.

## Patentansprüche

1. Expressionsvektor für die Expression eines Proteins, der eine Genexpressions-Regulationsstelle, die ein Elongationsfaktor-1α-Promotor ist, ein Gen, welche das stromabwärts davon gelegene Protein kodiert, eine interne ribosomale Eintrittstelle, die von dem 5'-untranslatierten Bereich des Encephalomyocarditis-Virus der Maus abgeleitet ist und die weiter stromabwärts davon gelegene Nukleotidsequenz nach SEQ ID NO: 4 oder SEQ ID NO: 6 aufweist, ein noch weiter stromabwärts davon gelegenes, eine Glutaminsynthetase kodierendes Gen und eine weitere Genexpressions-Regulationsstelle und ein stromabwärts davon gelegenes Puromycinresistenzgen aufweist.

2. Expressionsvektor nach Anspruch 1, wobei die weitere Genexpressions-Regulationsstelle ausgewählt ist aus der Gruppe bestehend aus einem vom Cytomegalovirus abgeleiteten Promotor, einem frühen Promotor von SV40 und einem Elongationsfaktor-1α-Promotor.

## Revendications

1. Vecteur d'expression pour l'expression d'une protéine, comprenant un site régulateur de l'expression d'un gène qui est un promoteur 1 alpha d'un facteur d'élongation, un gène encodant la protéine en aval de celui-ci, un site d'entrée du ribosome interne dérivé de la région 5' non traduite du virus de l'encéphalomyocardite de la souris et comprenant la séquence de nucléotides présentée sous la forme de SEQ ID NO : 4 ou SEQ ID NO : 6, plus en aval de celui-ci, un gène encodant une glutamine synthétase encore plus en aval de celui-ci, et un site supplémentaire régulateur de l'expression du gène et un gène de résistance à la puromycine en aval de celui-ci.

2. Vecteur d'expression selon la revendication 1, dans lequel le site supplémentaire régulateur de l'expression du gène est choisi dans le groupe constitué par le promoteur dérivé du cytomégalovirus, le promoteur précoce SV40, et le promoteur 1 alpha du facteur d'élongation.


### SEQUENCE LISTING

<110> JCR Pharmaceuticals. Co. Ltd.
<120> Novel Expression Vector
<130> GP151-PCT
<150> JP2010-250306 <151> 2010-11-08
<160> 33
<170> PatentIn version 3.5
<210> 1
   <211> 23
   <212> DNA
   <213> Murine encephalomyocarditis virus
<400> 1
   atgataatat ggccacaacc atg 23
<210> 2
   <211> 23
   <212> DNA
   <213> Artificial Sequence
<220>
   <223> modified Murine encephalomyocariditis virus
<220>
   <221> misc_feature
   <222> (9)..(11)
   <223> n is a, c, g, or t
<220>
   <221> misc_feature
   <222> (21) .. (23)
   <223> n is a, c, g, or t
<400> 2
   atgataatnn ngccacaacc nnn 23
<210> 3
   <211> 23
   <212> DNA
   <213> Artificial Sequence
<220>
   <223> modified Murine encephalomyocarditis virus
<220>
   <221> misc_feature
   <222> (9)..(11)
   <223> n is a, c, g, or t
<400> 3
   atgataatnn ngccacaacc atg 23
<210> 4
   <211> 23
   <212> DNA
   <213> Artificial Sequence
<220>
   <223> modified Murine encephalomyocarditis virus
<400> 4
   atgataagct tgccacaacc atg 23
<210> 5
   <211> 596
   <212> DNA
   <213> Murine encephalomyocarditis virus
<400> 5
   cccccccccc tctccctccc ccccccctaa cgttactggc cgaagccgct tggaataagg 60
   ccggtgtgcg tttgtctata tgttattttc caccatattg ccgtcttttg gcaatgtgag 120
   ggcccggaaa cctggccctg tcttcttgac gagcattcct aggggtcttt cccctctcgc 180
   caaaggaatg caaggtctgt tgaatgtcgt gaaggaagca gttcctctgg aagcttcttg 240
   aagacaaaca acgtctgtag cgaccctttg caggcagcgg aaccccccac ctggcgacag 300
   gtgcctctgc ggccaaaagc cacgtgtata agatacacct gcaaaggcgg cacaacccca 360
   gtgccacgtt gtgagttgga tagttgtgga aagagtcaaa tggctctcct caagcgtatt 420
   caacaagggg ctgaaggatg cccagaaggt accccattgt atgggatctg atctggggcc 480
   tcggtgcaca tgctttacat gtgtttagtc gaggttaaaa aaacgtctag gccccccgaa 540
   ccacggggac gtggttttcc tttgaaaaac acgatgataa tatggccaca accatg 596
<210> 6
   <211> 596
   <212> DNA
   <213> Artificial Sequence
<220>
   <223> modified Murine encephalomyocarditis virus
<400> 6
   cccccccccc tctccctccc ccccccctaa cgttactggc cgaagccgct tggaataagg 60
   ccggtgtgcg tttgtctata tgttattttc caccatattg ccgtcttttg gcaatgtgag 120
   ggcccggaaa cctggccctg tcttcttgac gagcattcct aggggtcttt cccctctcgc 180
   caaaggaatg caaggtctgt tgaatgtcgt gaaggaagca gttcctctgg aagcttcttg 240
   aagacaaaca acgtctgtag cgaccctttg caggcagcgg aaccccccac ctggcgacag 300
   gtgcctctgc ggccaaaagc cacgtgtata agatacacct gcaaaggcgg cacaacccca 360
   gtgccacgtt gtgagttgga tagttgtgga aagagtcaaa tggctctcct caagcgtatt 420
   caacaagggg ctgaaggatg cccagaaggt accccattgt atgggatctg atctggggcc 480
   tcggtgcaca tgctttacat gtgtttagtc gaggttaaaa aaacgtctag gccccccgaa 540
   ccacggggac gtggttttcc tttgaaaaac acgatgataa gcttgccaca accatg 596
<210> 7
   <211> 20
   <212> DNA
   <213> Artificial Sequence
<220>
   <223> Primer Hyg-Sfi5', synthetic sequence
<400> 7
   gaggccgcct cggcctctga 20
<210> 8
   <211> 29
   <212> DNA
   <213> Artificial Sequence
<220>
   <223> Primer Hyg-BstX3', synthetic sequence
<400> 8
   aaccatcgtg atgggtgcta ttcctttgc 29
<210> 9
   <211> 2216
   <212> DNA
   <213> Artificial Sequence
<220>
   <223> IRES-Hygr-mPGKpA, synthetic sequence
<220>
   <221> CDS
   <222> (716)..(1741)
<400> 9
   ctcgaggaat tcactccttc aggtgcaggc ttgcctatca gaaggtggtg gctggtgtgg 60
   ccaactggct cacaaatacc actgagatcg acggtatcga taagcttgat atcgaattcc 120
   gccccccccc cctctccctc ccccccccct aacgttactg gccgaagccg cttggaataa 180
   ggccggtgtg cgtttgtcta tatgttattt tccaccatat tgccgtcttt tggcaatgtg 240
   agggcccgga aacctggccc tgtcttcttg acgagcattc ctaggggtct ttcccctctc 300
   gccaaaggaa tgcaaggtct gttgaatgtc gtgaaggaag cagttcctct ggaagcttct 360
   tgaagacaaa caacgtctgt agcgaccctt tgcaggcagc ggaacccccc acctggcgac 420
   aggtgcctct gcggccaaaa gccacgtgta taagatacac ctgcaaaggc ggcacaaccc 480
   cagtgccacg ttgtgagttg gatagttgtg gaaagagtca aatggctctc ctcaagcgta 540
   ttcaacaagg ggctgaagga tgcccagaag gtaccccatt gtatgggatc tgatctgggg      600
   cctcggtgca catgctttac atgtgtttag tcgaggttaa aaaaacgtct aggccccccg      660
   aaccacgggg acgtggtttt cctttgaaaa acacgatgat aatatggcca caacc atg       718
                                                                Met
                                                                1
   aaa aag cct gaa ctc acc gcg acg tct gtc gag aag ttt ctg atc gaa        766
   Lys Lys Pro Glu Leu Thr Ala Thr Ser Val Glu Lys Phe Leu Ile Glu
               5                   10                  15
   aag ttc gac agc gtc tcc gac ctg atg cag ctc tcg gag ggc gaa gaa        814
   Lys Phe Asp Ser Val Ser Asp Leu Met Gln Leu Ser Glu Gly Glu Glu
           20                  25                  30
   tct cgt gct ttc agc ttc gat gta gga ggg cgt gga tat gtc ctg cgg        862
   Ser Arg Ala Phe Ser Phe Asp Val Gly Gly Arg Gly Tyr Val Leu Arg
       35                  40                  45
   gta aat agc tgc gcc gat ggt ttc tac aaa gat cgt tat gtt cat cgg        910
   Val Asn Ser Cys Ala Asp Gly Phe Tyr Lys Asp Arg Tyr Val His Arg
   50                  55                  60                  65
   cac ttt gca tcg gcc gcg ctc ccg att ccg gaa gtg ctt gac att ggg        958
   His Phe Ala Ser Ala Ala Leu Pro Ile Pro Glu Val Leu Asp Ile Gly
                   70                  75                  80
   gaa ttc agc gag agc ctg acc tat tgc atc tcc cgc cgt gca cag ggt       1006
   Glu Phe Ser Glu Ser Leu Thr Tyr Cys Ile Ser Arg Arg Ala Gln Gly
               85                  90                  95
   gtc acg ttg caa gac ctg cct gaa acc gaa ctg ccc gct gtt ctg cag       1054
   Val Thr Leu Gln Asp Leu Pro Glu Thr Glu Leu Pro Ala Val Leu Gln
           100                 105                 110
   ccg gtc gcg gag gcc atg gat gcg atc gct gcg gcc gat ctt agc cag       1102
   Pro Val Ala Glu Ala Met Asp Ala Ile Ala Ala Ala Asp Leu Ser Gln
       115                 120                 125
   acg agc ggg ttc ggc cca ttc gga ccg caa gga atc ggt caa tac act       1150
   Thr Ser Gly Phe Gly Pro Phe Gly Pro Gln Gly Ile Gly Gln Tyr Thr
   130                 135                 140                 145
   acg tgg cgt gat ttc ata tgc gcg att gct gat ccc cat gtg tat cac       1198
   Thr Trp Arg Asp Phe Ile Cys Ala Ile Ala Asp Pro His Val Tyr His
                   150                 155                 160
   tgg caa act gtg atg gac gac acc gtc agt gcg tcc gtc gcg cag gct       1246
   Trp Gln Thr Val Met Asp Asp Thr Val Ser Ala Ser Val Ala Gln Ala
               165                 170                 175
   ctc gat gag ctg atg ctt tgg gcc gag gac tgc ccc gaa gtc cgg cac       1294
   Leu Asp Glu Leu Met Leu Trp Ala Glu Asp Cys Pro Glu Val Arg His
           180                 185                 190
   ctc gtg cac gcg gat ttc ggc tcc aac aat gtc ctg acg gac aat ggc       1342
   Leu Val His Ala Asp Phe Gly Ser Asn Asn Val Leu Thr Asp Asn Gly
       195                 200                 205
   cgc ata aca gcg gtc att gac tgg agc gag gcg atg ttc ggg gat tcc       1390
   Arg Ile Thr Ala Val Ile Asp Trp Ser Glu Ala Met Phe Gly Asp Ser
   210                 215                 220                 225
   caa tac gag gtc gcc aac atc ttc ttc tgg agg ccg tgg ttg gct tgt        1438
   Gln Tyr Glu Val Ala Asn Ile Phe Phe Trp Arg Pro Trp Leu Ala Cys
                   230                 235                 240
   atg gag cag cag acg cgc tac ttc gag cgg agg cat ccg gag ctt gca        1486
   Met Glu Gln Gln Thr Arg Tyr Phe Glu Arg Arg His Pro Glu Leu Ala
               245                 250                 255
   gga tcg ccg cgg ctc cgg gcg tat atg ctc cgc att ggt ctt gac caa        1534
   Gly Ser Pro Arg Leu Arg Ala Tyr Met Leu Arg Ile Gly Leu Asp Gln
           260                 265                 270
   ctc tat cag agc ttg gtt gac ggc aat ttc gat gat gca gct tgg gcg        1582
   Leu Tyr Gln Ser Leu Val Asp Gly Asn Phe Asp Asp Ala Ala Trp Ala
       275                 280                 285
   cag ggt cga tgc gac gca atc gtc cga tcc gga gcc ggg act gtc ggg        1630
   Gln Gly Arg Cys Asp Ala Ile Val Arg Ser Gly Ala Gly Thr Val Gly
   290                 295                 300                 305
   cgt aca caa atc gcc cgc aga agc gcg gcc gtc tgg acc gat ggc tgt        1678
   Arg Thr Gln Ile Ala Arg Arg Ser Ala Ala Val Trp Thr Asp Gly Cys
                   310                 315                 320
   gta gaa gta ctc gcc gat agt gga aac cga cgc ccc agc act cgt ccg        1726
   Val Glu Val Leu Ala Asp Ser Gly Asn Arg Arg Pro Ser Thr Arg Pro
               325                 330                 335
   agg gca aag gaa tag tcgagaaatt gatgatctat taagcaataa agacgtccac        1781
   Arg Ala Lys Glu
           340
   taaaatggaa gtttttcctg tcatactttg ttaagaaggg tgagaacaga gtacctacat      1841
   tttgaatgga aggattggag ctacgggggt gggggtgggg tgggattaga taaatgcctg      1901
   ctctttactg aaggctcttt actattgctt tatgataatg tttcatagtt ggatatcata      1961
   atttaaacaa gcaaaaccaa attaagggcc agctcattcc tccactcacg atctatagat      2021
   ccactagctt ggcgtaatca tggtcatagc tgtttcctgt gtgaaattgt tatccgctca      2081
   caattccaca caacatacga gccggaagca taaagtgtaa agcctggggt gcctaatgag      2141
   tgagctaact cacattaatt gcgttgcgct cactgcccgc tttccagtcg ggaaacctgt      2201
   cgtgccagcg gatcc                                                       2216
<210> 10
   <211> 341
   <212> PRT
   <213> Artificial Sequence
<220>
   <223> Synthetic Construct
<400> 10
   Met Lys Lys Pro Glu Leu Thr Ala Thr Ser Val Glu Lys Phe Leu Ile
   1               5                   10                  15
   Glu Lys Phe Asp Ser Val Ser Asp Leu Met Gln Leu Ser Glu Gly Glu
               20                  25                  30
   Glu Ser Arg Ala Phe Ser Phe Asp Val Gly Gly Arg Gly Tyr Val Leu
           35                  40                  45
   Arg Val Asn Ser Cys Ala Asp Gly Phe Tyr Lys Asp Arg Tyr Val His
       50                  55                  60
   Arg His Phe Ala Ser Ala Ala Leu Pro Ile Pro Glu Val Leu Asp Ile
   65                  70                  75                  80
   Gly Glu Phe Ser Glu Ser Leu Thr Tyr Cys Ile Ser Arg Arg Ala Gln
                   85                  90                  95
   Gly Val Thr Leu Gln Asp Leu Pro Glu Thr Glu Leu Pro Ala Val Leu
               100                 105                 110
   Gln Pro Val Ala Glu Ala Met Asp Ala Ile Ala Ala Ala Asp Leu Ser
           115                 120                 125
   Gln Thr Ser Gly Phe Gly Pro Phe Gly Pro Gln Gly Ile Gly Gln Tyr
       130                 135                 140
   Thr Thr Trp Arg Asp Phe Ile Cys Ala Ile Ala Asp Pro His Val Tyr
   145                 150                 155                 160
   His Trp Gln Thr Val Met Asp Asp Thr Val Ser Ala Ser Val Ala Gln
                   165                 170                 175
   Ala Leu Asp Glu Leu Met Leu Trp Ala Glu Asp Cys Pro Glu Val Arg
               180                 185                 190
   His Leu Val His Ala Asp Phe Gly Ser Asn Asn Val Leu Thr Asp Asn
           195                 200                 205
   Gly Arg Ile Thr Ala Val Ile Asp Trp Ser Glu Ala Met Phe Gly Asp
       210                 215                 220
   Ser Gln Tyr Glu Val Ala Asn Ile Phe Phe Trp Arg Pro Trp Leu Ala
   225                 230                 235                 240
   Cys Met Glu Gln Gln Thr Arg Tyr Phe Glu Arg Arg His Pro Glu Leu
                   245                 250                 255
   Ala Gly Ser Pro Arg Leu Arg Ala Tyr Met Leu Arg Ile Gly Leu Asp
                   260                 265                 270
   Gln Leu Tyr Gln Ser Leu Val Asp Gly Asn Phe Asp Asp Ala Ala Trp
           275                 280                 285
   Ala Gln Gly Arg Cys Asp Ala Ile Val Arg Ser Gly Ala Gly Thr Val
       290                 295                 300
   Gly Arg Thr Gln Ile Ala Arg Arg Ser Ala Ala Val Trp Thr Asp Gly
   305                 310                 315                     320
   Cys Val Glu Val Leu Ala Asp Ser Gly Asn Arg Arg Pro Ser Thr Arg
                   325                 330                 335
   Pro Arg Ala Lys Glu
               340
<210> 11
   <211> 53
   <212> DNA
   <213> Artificial Sequence
<220>
   <223> Primer IRES5', synthetic sequence
<400> 11
   caactcgagc ggccgccccc cccccctctc cctccccccc ccctaacgtt act 53
<210> 12
   <211> 22
   <212> DNA
   <213> Artificial Sequence
<220>
   <223> Primer IRES3', synthetic sequence
<400> 12
   caagaagctt ccagaggaac tg 22
<210> 13
   <211> 30
   <212> DNA
   <213> Artificial Sequence
<220>
   <223> Primer mPGKP5', synthetic sequence
<400> 13
   gcgagatctt accgggtagg ggaggcgctt 30
<210> 14
   <211> 30
   <212> DNA
   <213> Artificial Sequence
<220>
   <223> Primer mPGKP3', synthetic sequence
<400> 14
   gaggaattcg atgatcggtc gaaaggcccg 30
<210> 15
   <211> 532
   <212> DNA
   <213> Artificial Sequence
<220>
   <223> mPGKp, synthetic sequence
<400> 15
   gcgagatctt accgggtagg ggaggcgctt ttcccaaggc agtctggagc atgcgcttta 60
   gcagccccgc tgggcacttg gcgctacaca agtggcctct ggcctcgcac acattccaca 120
   tccaccggta ggcgccaacc ggctccgttc tttggtggcc ccttcgcgcc accttctact 180
   cctcccctag tcaggaagtt cccccccgcc ccgcagctcg cgtcgtgcag gacgtgacaa 240
   atggaagtag cacgtctcac tagtctcgtg cagatggaca gcaccgctga gcaatggaag 300
   cgggtaggcc tttggggcag cggccaatag cagctttgct ccttcgcttt ctgggctcag 360
   aggctgggaa ggggtgggtc cgggggcggg ctcaggggcg ggctcagggg cggggcgggc 420
   gcccgaaggt cctccggagg cccggcattc tgcacgcttc aaaagcgcac gtctgccgcg 480
   ctgttctcct cttcctcatc tccgggcctt tcgaccgatc atcgaattcc tc 532
<210> 16
   <211> 36
   <212> DNA
   <213> Artificial Sequence
<220>
   <223> Primer GS5', synthetic sequence
<400> 16
   aatatggcca caaccatggc gacctcagca agttcc 36
<210> 17
   <211> 38
   <212> DNA
   <213> Artificial Sequence
<220>
   <223> Primer GS3', synthetic sequence
<400> 17
   ggaggatccc tcgagttagt ttttgtattg gaagggct 38
<210> 18
   <211> 28
   <212> DNA
   <213> Artificial Sequence
<220>
   <223> Primer puro5', synthetic sequence
<400> 18
   gcttaagatg accgagtaca agcccacg 28
<210> 19
   <211> 30
   <212> DNA
   <213> Artificial Sequence
<220>
   <223> Primer puro3', synthetic sequence
<400> 19
   cccatcgtga tggtcaggca ccgggcttgc 30
<210> 20
   <211> 620
   <212> DNA
   <213> Artificial Sequence
<220>
   <223> Puromycin
<220>
   <221> CDS
   <222> (8)..(607)
<400> 20
   gcttaag atg acc gag tac aag ccc acg gtg cgc ctc gcc acc cgc gac            49
           Met Thr Glu Tyr Lys Pro Thr Val Arg Leu Ala Thr Arg Asp
           1               5                   10
   gac gtc ccc agg gcc gta cgc acc ctc gcc gcc gcg ttc gcc gac tac            97
   Asp Val Pro Arg Ala Val Arg Thr Leu Ala Ala Ala Phe Ala Asp Tyr
   15                  20                  25                  30
   ccc gcc acg cgc cac acc gtc gat ccg gac cgc cac atc gag cgg gtc           145
   Pro Ala Thr Arg His Thr Val Asp Pro Asp Arg His Ile Glu Arg Val
                   35                  40                  45
   acc gag ctg caa gaa ctc ttc ctc acg cgc gtc ggg ctc gac atc ggc           193
   Thr Glu Leu Gln Glu Leu Phe Leu Thr Arg Val Gly Leu Asp Ile Gly
               50                  55                  60
   aag gtg tgg gtc gcg gac gac ggc gcc gcg gtg gcg gtc tgg acc acg           241
   Lys Val Trp Val Ala Asp Asp Gly Ala Ala Val Ala Val Trp Thr Thr
           65                  70                  75
   ccg gag agc gtc gaa gcg ggg gcg gtg ttc gcc gag atc ggc ccg cgc           289
   Pro Glu Ser Val Glu Ala Gly Ala Val Phe Ala Glu Ile Gly Pro Arg
       80                  85                  90
   atg gcc gag ttg agc ggt tcc cgg ctg gcc gcg cag caa cag atg gaa           337
   Met Ala Glu Leu Ser Gly Ser Arg Leu Ala Ala Gln Gln Gln Met Glu
   95                  100                 105                 110
   ggc ctc ctg gcg ccg cac cgg ccc aag gag ccc gcg tgg ttc ctg gcc           385
   Gly Leu Leu Ala Pro His Arg Pro Lys Glu Pro Ala Trp Phe Leu Ala
                   115                 120                 125
   acc gtc ggc gtc tcg ccc gac cac cag ggc aag ggt ctg ggc agc gcc           433
   Thr Val Gly Val Ser Pro Asp His Gln Gly Lys Gly Leu Gly Ser Ala
               130                 135                 140
   gtc gtg ctc ccc gga gtg gag gcg gcc gag cgc gcc ggg gtg ccc gcc           481
   Val Val Leu Pro Gly Val Glu Ala Ala Glu Arg Ala Gly Val Pro Ala
           145                 150                 155
   ttc ctg gag acc tcc gcg ccc cgc aac ctc ccc ttc tac gag cgg ctc           529
   Phe Leu Glu Thr Ser Ala Pro Arg Asn Leu Pro Phe Tyr Glu Arg Leu
       160                 165                 170
   ggc ttc acc gtc acc gcc gac gtc gag gtg ccc gaa gga ccg cgc acc           577
   Gly Phe Thr Val Thr Ala Asp Val Glu Val Pro Glu Gly Pro Arg Thr
   175                 180                 185                 190
   tgg tgc atg acc cgc aag ccc ggt gcc tga ccatcacgat ggg                    620
   Trp Cys Met Thr Arg Lys Pro Gly Ala
                   195
<210> 21
   <211> 199
   <212> PRT
   <213> Artificial Sequence
<220>
   <223> Synthetic Construct
<400> 21
   Met Thr Glu Tyr Lys Pro Thr Val Arg Leu Ala Thr Arg Asp Asp Val
   1               5                   10                  15
   Pro Arg Ala Val Arg Thr Leu Ala Ala Ala Phe Ala Asp Tyr Pro Ala
               20                  25                  30
   Thr Arg His Thr Val Asp Pro Asp Arg His Ile Glu Arg Val Thr Glu
           35                  40                  45
   Leu Gln Glu Leu Phe Leu Thr Arg Val Gly Leu Asp Ile Gly Lys Val
       50                  55                  60
   Trp Val Ala Asp Asp Gly Ala Ala Val Ala Val Trp Thr Thr Pro Glu
   65                  70                  75                  80
   Ser Val Glu Ala Gly Ala Val Phe Ala Glu Ile Gly Pro Arg Met Ala
                   85                  90                  95
   Glu Leu Ser Gly Ser Arg Leu Ala Ala Gln Gln Gln Met Glu Gly Leu
               100                 105                 110
   Leu Ala Pro His Arg Pro Lys Glu Pro Ala Trp Phe Leu Ala Thr Val
           115                 120                 125
   Gly Val Ser Pro Asp His Gln Gly Lys Gly Leu Gly Ser Ala Val Val
       130                 135                 140
   Leu Pro Gly Val Glu Ala Ala Glu Arg Ala Gly Val Pro Ala Phe Leu
   145                 150                 155                 160
   Glu Thr Ser Ala Pro Arg Asn Leu Pro Phe Tyr Glu Arg Leu Gly Phe
                   165                 170                 175
   Thr Val Thr Ala Asp Val Glu Val Pro Glu Gly Pro Arg Thr Trp Cys
               180                 185                 190
   Met Thr Arg Lys Pro Gly Ala
           195
<210> 22
   <211> 44
   <212> DNA
   <213> Artificial Sequence
<220>
   <223> Primer SV40polyA5', synthetic sequence
<400> 22
   caacaagcgg ccgccctcga gttcccttta gtgagggtta atgc 44
<210> 23
   <211> 24
   <212> DNA
   <213> Artificial Sequence
<220>
   <223> Primer SV40polyA3', synthetic sequence
<400> 23
   cccctgaacc tgaaacataa aatg 24
<210> 24
   <211> 25
   <212> DNA
   <213> Artificial Sequence
<220>
   <223> Primer mIRES-GS5', synthetic sequence
<400> 24
   acacgatgat aagcttgcca caacc 25
<210> 25
   <211> 21
   <212> DNA
   <213> Artificial Sequence
<220>
   <223> Primer mIRES-GS3', synthetic sequence
<400> 25
   ctccacgata tccctgccat a 21
<210> 26
   <211> 23
   <212> DNA
   <213> Artificial Sequence
<220>
   <223> Primer SV40polyA5'-2, synthetic sequence
<400> 26
   actaactcga gttcccttta gtg 23
<210> 27
   <211> 26
   <212> DNA
   <213> Artificial Sequence
<220>
   <223> Primer SV40polyA3'-2, synthetic sequence
<400> 27
   aacggatcct tatcggattt taccac 26
<210> 28
   <211> 47
   <212> DNA
   <213> Artificial Sequence
<220>
   <223> Primer hGBA5', synthetic sequence
<400> 28
   gcaatacgcg tccgccacca tggagttttc aagtccttcc agagagg 47
<210> 29
   <211> 41
   <212> DNA
   <213> Artificial Sequence
<220>
   <223> Primer hGBA3', synthetic sequence
<400> 29
   ggacgcggcc gcgagctctc actggcgacg ccacaggtag g 41
<210> 30
   <211> 39
   <212> DNA
   <213> Artificial Sequence
<220>
   <223> Primer hEPO5', synthetic sequence
<400> 30
   aagacgcgtc gccaccatgg gggtgcacga atgtcctgc 39
<210> 31
   <211> 35
   <212> DNA
   <213> Artificial Sequence
<220>
   <223> Primer hEPO3', synthetic sequence
<400> 31
   aagagcggcc gctcatctgt cccctgtcct gcagg 35
<210> 32
   <211> 23
   <212> DNA
   <213> Artificial Sequence
<220>
   <223> modified Murine encephalomyocariditis virus
<220>
   <221> misc_feature
   <222> (8)..(11)
   <223> n is a, c, g, or t
<220>
   <221> misc_feature
   <222> (21) .. (23)
   <223> n is a, c, g, or t
<400> 32
   atgataannn ngccacaacc nnn 23
<210> 33
   <211> 23
   <212> DNA
   <213> Artificial Sequence
<220>
   <223> modified Murine encephalomyocarditis virus
<220>
   <221> misc_feature
   <222> (8)..(11)
   <223> n is a, c, g, or t
<400> 33
   atgataannn ngccacaacc atg 23